# EUROPEAN PATENT SPECIFICATION

(11) **EP 4 159 200 B1**
(45) Date of publication and mention of the grant of the patent: **13.08.2025**
(21) Application number: 21841818.4
(22) Date of filing: 19.05.2021
(51) Int. Cl.: A61K 9/16, A61K 31/4439, A61K 47/32, A61P 31/14

(54) **PHARMACEUTICAL COMPOSITION AND PREPARATION METHOD**
PHARMAZEUTISCHE ZUSAMMENSETZUNG UND HERSTELLUNGSVERFAHREN
COMPOSITION PHARMACEUTIQUE ET SA MÉTHODE DE PRÉPARATION

(30) Priority: 17.07.2020 CN 202010693600
(43) Date of publication of application: 05.04.2023
(73) Proprietor: Jiangsu Kanion Pharmaceutical Co., Ltd., Jiangsu 222047 (CN)
(72) Inventor: XIAO, Wei, Lianyungang, Jiangsu 222047 (CN); ZHANG, Hui, Lianyungang, Jiangsu 222047 (CN); GUO, Qingming, Lianyungang, Jiangsu 222047 (CN); WANG, Zhenzhong, Lianyungang, Jiangsu 222047 (CN); GU, Shasha, Lianyungang, Jiangsu 222047 (CN); HE, Xiaolian, Lianyungang, Jiangsu 222047 (CN); DONG, Xuehong, Lianyungang, Jiangsu 222047 (CN); WANG, Yanqiu, Lianyungang, Jiangsu 222047 (CN)
(74) Representative: Kurig, Thomas
(86) International application number: PCT/CN2021/094653
(87) International publication number: WO 2022/012152

(56) References cited:
- EP-A1- 3 138 840
- WO-A1-2015/165340
- WO-A1-2015/165340
- WO-A1-2017/071569
- WO-A1-2017/071569
- CA-A1- 3 098 006
- CN-A- 101 278 915
- CN-A- 101 862 333
- CN-A- 103 893 247
- CN-A- 103 893 258

## Description

### Technical Field

The present invention belongs to the field of pharmaceutical preparations, and particularly relates to a pharmaceutical composition suitable for use in the treatment of child hand-foot-and-mouth disease, and a preparation method thereof.

### Background

Hand-foot-and-mouth disease (HFMD) is a global infectious disease caused by enterovirus. The main pathogenic viruses include enterovirus 71 (EV71), Coxsachie A16 (CoxA16) and other enteroviruses, where EV71 is the most harmful. The hand-foot-and-mouth disease is more common in infants and children, especially children under 5 years old, but less common in adults. However, patients with immunodeficiency may also be infected. At onset, blisters on hands, feet and mouth are the typical features. In addition, the disease is accompanied by symptoms such as fever, headache, sore throat and rash, etc. In severe cases, myocarditis, pulmonary edema, aseptic meningitis and even fatal pulmonary embolism or massive hemorrhage may occur. Individual severe children's conditions develop rapidly and the mortality rate is high.

The compound has been disclosed at least in two prior art documents [CN105085512A and CN106661009A]. Both of the documents have disclosed the structure and use of the compound to treat hand foot mouth disease caused by enterovirus 71 and the preparation method thereof.

Specifically, the last chemical structure in the ninth page of the CN106661009A is the structure of the compound and its Embodiment 75F (100 pages) discloses the preparation method and nuclear magnetic hydrogen spectrum of the compound. The last chemical structure of para [0055] discloses the structure of the compound, and the preparation method and nuclear magnetic hydrogen spectrum of the compound are disclosed in [1168] - [1171] paras of CN105085512A.

WO2017071569A1 discloses a salt form and crystal form of a compound 1, preparation method thereof and intermediate:

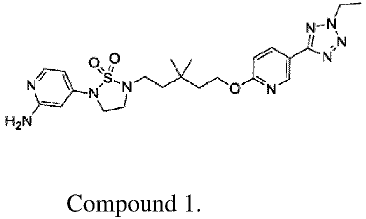

EP3138840A1 discloses a novel anti-enterovirus 71 (EV71) 1, 2, 5-thiadiazolidine-1,1-dioxide derivative or a pharmaceutically acceptable salt thereof, and specifically, a compound represented by formula (II) or a pharmaceutically acceptable salt thereof:

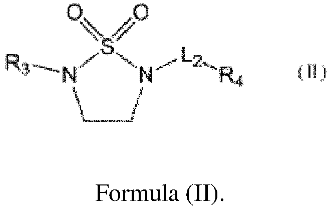

WO2015165340A1 discloses a novel anti-enterovirus 71 (EV71) 1,2,5-thiadiazolidine-1,1-dioxide derivative or a pharmaceutically acceptable salt thereof, and specifically, a compound represented by formula (II) or a pharmaceutically acceptable salt thereof:

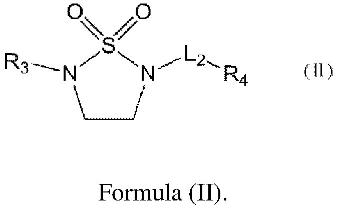

### Summary

An objective of the present invention is to provide a pharmaceutical composition with high stability and rapid dissolution and suitable for treating child hand-foot-and-mouth disease, and a preparation method thereof. Specifically, the pharmaceutical composition provided by the present invention includes:
an active ingredient, including 2-(2-aminopyridine-4-yl)-5-(5-((5-(2-ethyl-2H-tetrazole-5-yl)pyridine-2-yl)-oxy)-3,3-amyldimethyl)-1,2,5-thiadiazolidin 1,1-dioxide or pharmaceutically acceptable salt thereof;
the pharmaceutical composition is characterized by further comprising auxiliary materials, including a diluent, a suspending agent and a solubilizer;
the diluent comprising one or more of sucrose, mannitol, sorbitol and xylitol, the suspending agent comprising povidone, and the solubilizer comprising poloxamer 188 and/or polysorbate 80;
wherein, the active ingredient accounts for 1.0% to 3.0% of a total weight of the pharmaceutical composition, the diluent accounts for 60% to 98% of the total weight of the pharmaceutical composition, the suspending agent accounts for 0.3% to 2.0% of the total weight of the pharmaceutical composition, and the solubilizer accounts for 0.5% to 2.0% of a total weight of the pharmaceutical composition.

Further, the suspending agent may further include one or more of carboxymethylcellulose sodium, carrageenan or crospovidone (CL-M).

Specifically, the pharmaceutically acceptable salt includes hydrochloride, sulfate, citrate, benzenesulfonate, hydrobromide, hydrofluoric acid, phosphate, acetate, propionate, succinate, oxalate, malate, fumarate, maleate, tartrate or trifluoroacetate.

Optionally, the povidone is povidone K30 or povidone K90.

Further, the content of the suspending agent povidone K90 is 0.5 to 1.5%.

Further, the content of the suspending agent crospovidone (CL-M) is 0.5% to 2.0% of a total weight of the composition, preferably, 0.8 to 1.5%.

Further,the solubilizer preferably accounts for 0.8 to 1.5% of a total weight of the pharmaceutical composition.

Preferably, the diluent includes mannitol and sorbitol; and the weight ratio of the mannitol to the sorbitol is 1:1~ 3:1.

Further, the auxiliary materials further include essence; the essence includes one or more of orange essence, strawberry essence and banana essence; and the essence preferably accounts for ≤ 1.0% of a total weight of the pharmaceutical composition, preferably, 0.1% to 0.5%.

Further, the specific weight of each bag of active ingredient may be 0.1 mg to 200 mg, preferably, 1 mg to 100 mg, more preferably, 10 mg to 100 mg, most preferably, 50 mg.

Further, the diluent may account for 80% to 95% of a total weight of the pharmaceutical composition.

Preferably, the pharmaceutical composition provided by the present invention includes: 1 part of compound A, 26 parts of mannitol, 12 parts of sorbitol and 0.2 parts to 0.3 parts of povidone K30 or povidone K90.

Further, the pharmaceutical composition may further include: 0.4 parts of poloxamer 188.

Further, the pharmaceutical composition may further include: 0.4 parts of crospovidone (CL-M).

Further, the pharmaceutical composition may further include: 0.08 parts of strawberry essence.

Specifically, the pharmaceutical composition is a granule prepared from the active ingredient and the auxiliary materials through mixing, granulation, drying, granule arrangement, total mixing and packaging.

The present invention further provides a use of the pharmaceutical composition for treating child hand-foot-and-mouth disease.

The present invention further provides a method for preparing the pharmaceutical composition as described in any one of the above. The method includes:
a) obtaining an adhesive by the suspending agent, the solubilizer and ethanol;;
b) mixing the active ingredient and the diluent, and adding the adhesive for granulation;
c) performing dynamic drying; and
d) performing granule arrangement and total mixing.

Further, the method may include:
a) adding 0.4 parts of poloxamer 188 and 0.3 parts of povidone K90 into a 80% ethanol solution for uniform mixing to serve as the adhesive;
b) weighing 2-(2-aminopyridine-4-yl)-5-(5-((5-(2-ethyl-2H-tetrazole-5-yl)pyridine-2-yl)-oxy)-3,3-amyldimethyl)-1,2,5-thiadiazolidin 1,1-dioxide hydrochloride, mannitol, sorbitol and crospovidone (CL-M) according to a weight ratio of 1:26:12:0.4 by part, mixing the materials uniformly and then adding the adhesive to obtain a soft material, and performing sieving with a 841 micrometer (20-mesh) sieve for granulation:
c) performing drying at 50°C; and
d) performing granule arrangement with a 841 micrometer (20-mesh) sieve, adding essence into the granules after granule arrangement for total mixing, and packaging.

According to the present invention, the pharmaceutical composition with rapid dissolution and high stability is finally obtained by screening the types and the dosage ratio of the auxiliary materials; and the pharmaceutical composition is simple in preparation process and more suitable for industrialized mass production.

### Detailed Description of Embodiments

An active substance (compound A: 2-(2-aminopyridine-4-yl)-5-(5-((5-(2-ethyl-2H-tetrazole-5-yl)pyridine-2-yl)-oxy)-3,3-amyldimethyl)-1,2,5-thiadiazolidin 1,1-dioxide hydrochloride) of the present invention can lock viruses by entering a pocket cavity of an EV71 virus nucleocapsid to prevent a viral capsid from decomposing and releasing virus RNA, thereby inhibiting further replication of the virus. The structural formula is as follows: 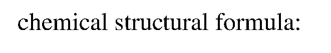 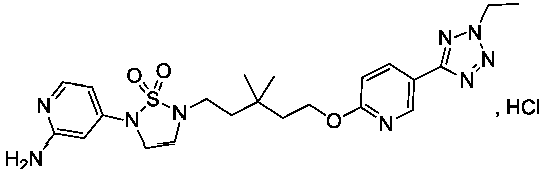
molecular formula: C₂₂H₃₁N₉O₃S.HCl
molecular weight: 538.07

To improve the solubility of insoluble medicines, surfactants, acid-base regulators and the like are mostly used in the prior art. According to the present invention, it is found in the preliminary test process that a compound A and an acidic regulator (citric acid or tartaric acid) can realize complete dissolution of the compound A; upon further study, it is found that when the adding amount of the acid is 0.2 to 1.0 that of the compound A, the solubility of the compound A in hot water can be improved. However, the granules obtained in the preliminary study process can solve the problem of solubility, but the stability is insufficient. Therefore, how to improve the solubility of the active substance provided by the present invention and ensure the stability has become a difficult point in a medicine technology.

In view of this, the present invention provides other preparation forms of the pharmaceutical composition suitable for treating child hand-foot-and-mouth disease, thereby solving the problem of the solubility and the stability of the compound by selecting appropriate auxiliary materials.

The present invention is further described below by the following embodiments and test examples. The embodiments and test examples are only used to illustrate the objective, but not to limit the scope of the present invention, which is defined by the appended claims.

In order to enable those skilled in the art to more clearly understand the technical solution of the present application, the technical solution of the present application will be described in detail below with reference to specific embodiments.

The test materials used in the embodiments of the present invention are all conventional test materials in the field, which can be purchased through commercial channels or can be prepared by methods in the prior art.

### Reference Example 1:

1 part of compound A, 26 parts of mannitol, 12 parts of sorbitol, 0.2 parts of carboxymethylcellulose sodium and 0.08 parts of strawberry essence;

### Reference Example 2:

1 part of compound A, 26 parts of mannitol, 12 parts of sorbitol, 0.2 parts of hydroxypropyl methylcellulose and 0.08 parts of strawberry essence;

### Reference Example 3:

: 1 part of compound A, 26 parts of mannitol, 12 parts of sorbitol, 0.2 parts of povidone K30 and 0.08 parts of strawberry essence;

### Reference Example 4:

1 part of compound A, 26 parts of mannitol, 12 parts of sorbitol, 0.2 parts of povidone K90 and 0.08 parts of strawberry essence;

### Example 5:

: 1 part of compound A, 26 parts of mannitol, 12 parts of sorbitol, 0.2 parts of povidone K90, 0.4 parts of poloxamer 188 and 0.08 parts of strawberry essence;

### Example 6:

1 part of compound A, 26 parts of mannitol, 12 parts of sorbitol, 0.3 parts of povidone K90, 0.4 parts of poloxamer 188 and 0.08 parts of strawberry essence;

### Example 7:

: 1 part of compound A, 26 parts of mannitol, 12 parts of sorbitol, 0.3 parts of povidone K90, 0.4 parts of poloxamer 188, 0.4 parts of crospovidone (CL-M) and 0.08 parts of strawberry essence;

### Reference Example 8:

: 1 part of compound A, 26 parts of mannitol, 12 parts of sorbitol, 0.2 parts of povidone K90, 0.1 part of carboxymethylcellulose sodium and 0.08 parts of strawberry essence; and

### Example 9:

1 part of compound A, 26 parts of mannitol, 12 parts of sorbitol, 0.2 parts of povidone K90, 0.3 parts of crospovidone (CL-M), 0.4 parts of polysorbate 80 and 0.08 parts of strawberry essence.

### Preparation processes of Examples 1-9:

① carboxymethylcellulose sodium/hydroxypropyl methylcellulose/povidone K30/povidone K90/poloxamer 188/polysorbate 80 were weighed according to a formula and were dissolved into 80% ethanol to prepare an adhesive.
② A compound A and auxiliary materials mannitol/sorbitol/crospovidone (CL-M) were mixed uniformly, and the adhesive was added to prepare a soft material.
③ Granulation was performed with a 841 micrometer (20-mesh) sieve.
④ Drying was performed at 50°C and the water content was controlled to be less than 2.0%.
⑤ Granule arrangement was performed with a 841 micrometer (20-mesh) sieve, and fine powder was removed with a 177 micrometer (20-mesh) sieve.
⑥ Essence was added and then total mixing was performed.
⑦ Granules after total mixing were collected.
⑧ Internal packaging was performed.

Comparative example 1: 1 part of compound A, 38 parts of sucrose, 0.2 parts of tartaric acid and 0.08 parts of strawberry essence.

A preparation process of Comparative Example 1:
① tartaric acid was weighed and dissolved in 80% ethanol to prepare an adhesive.
② A compound A and other auxiliary materials (except the tartaric acid and essence) were mixed uniformly, and the adhesive was added to prepare a soft material.
③ Granulation was performed with a 841 micrometer (20-mesh) sieve.
④ Drying was performed at 50°C and the water content was controlled to be less than 2.0%.
⑤ Granule arrangement was performed with a 841 micrometer (20-mesh) sieve, and fine powder was removed with a 177 micrometer (80-mesh) sieve.
⑥ Essence was added and then total mixing was performed.
⑦ Granules after total mixing were collected.
⑧ Internal packaging was performed.

Comparative example 2: 1 part of compound A, 38 parts of sucrose, 0.6 parts of tartaric acid and 0.08 parts of strawberry essence.

A preparation process of Comparative Example 2 is the same as that of Comparative example 1.

Comparative example 3: 1 part of compound A, 38 parts of sucrose, 0.6 parts of tartaric acid, 0.4 parts of polysorbate 80 and 0.08 parts of strawberry essence.

A preparation process of Comparative Example 3 is the same as that of Comparative example 1.

### 1. Solubility investigation

Granules prepared by the embodiments and the comparative examples were added into hot water to investigate the solubility. The results are shown in Table 1.

**Table 1 The investigation results of the solubility**

| Cases | Granule Categories | Dissolution Time | Dissolution Phenomenon |
|---|---|---|---|
| Reference Example 1 | Suspension granule | / | Flocculent precipitate |
| Reference Example 2 | Suspension granule | / | Flocculent precipitate |
| Reference Example 3 | Suspension granule | 18 s | The solution is a milky solution, stands for 3 min for settlement, and is shaken for 1 min, which is dispersed non-uniformly. (Heat preservation at 40°C) |
| Reference Example 4 | Suspension granule | 35 s | The solution is a milky solution, stands for 8 min for settlement, and is shaken for 1 min, which has low dispersibility. (Heat preservation at 40°C) |
| Example 5 | Suspension granule | 15 s | The solution is a semi-transparent and uniform milky solution, stands for 10 min for settlement, and is shaken for 1 min, which has low dispersibility. (Heat preservation at 40°C) |
| Example 6 | Suspension granule | 10 s | The solution is a semi-transparent and uniform milky solution, stands for 30 min for settlement, and is shaken for 1 min, which is dispersed uniformly. (Heat preservation at 40°C) |
| Example 7 | Suspension granule | 7 s | The solution is a semi-transparent and uniform milky solution, stands for 45 min for settlement, and is shaken for 20 s, which is dispersed uniformly. (Heat preservation at 40°C) |
| Reference Example 8 | Suspension granule | 35 s | The solution is a milky solution and is shaken for 1 min, which has low dispersibility. (Heat preservation at 40°C) |
| Example 9 | Suspension granule | 12 s | The solution is a semi-transparent and uniform milky solution, stands for 45 min for settlement, and is shaken for 20 s, which is dispersed uniformly and is slightly stink. (Heat preservation at 40°C) |
| Comparative example 1 | Soluble granule | 16 s | The solution is completely dissolved and is slightly turbid. |
| Comparative example 2 | Soluble granule | 8 s | The solution is completely dissolved, and is clear and transparent. |
| Comparative example 3 | Soluble granule | 6 s | The solution is completely dissolved, and is clear and transparent. |

According to the investigation results of the solubility, flocculent precipitates are found in Reference Examples 1 and 2; suspension milky solutions are found in the examples or comparative examples in which povidone is added; semi-transparent milky solutions dispersed uniformly are found in Example 6 and Example 7; the solution in Example 7 can be dispersed rapidly and uniformly through shaking after settlement; and polysorbate 80 is added in Example 9 and the solution is slightly stink, and Example 7 is finally preferred in view of the odor.

### 2. Stability investigation

According to the guide principle of stability test of crude medicines and pharmaceutical preparations in Appendix XIXC of PHARMACOPOEIA OF THE PEOPLE'S REPUBLIC OF CHINA, 2015, Volume II, in the present invention, suspension granules in Examples 6 and 7, and soluble granules in Comparative Examples 2 and 3 were investigated by accelerated stability test and long-term stability test (Example 7 and Comparative Example 3 were merely listed below).

The condition of the accelerated stability test: the pharmaceutical compositions prepared in Example 7 and Comparative example 3 were respectively packaged according to a composite film for packaging a polyester/aluminum/polyethylene medicine product, and stood for 6 months under the temperature of 40°C and the relative humidity of 75%, sampling was performed in the 0^{th}, 1^{st}, 3^{rd} and 6^{th} months during the test, and inspection was performed according to stability investigation items. The inspection results of the accelerated test of "related substances" are shown in Table 2. The results show that impurities in Comparative example 3 are higher than those in Example 7, which indicates that the solubility of the formula in the comparative example meets the requirement, but the stability is insufficient. Similarly, the change of related substances of the suspension granules in Example 6 is much smaller than those in Comparative examples 2 and 3.

**Table 2 Inspection results of related substances of accelerated stability test**

| Inspection Items | | Related Substances (%) | |
|---|---|---|---|
| | | Example 7 | Comparative example 3 |
| 0^{th} month | Largest single Impurity | 0.05 | 0.07 |
| | Total impurities | 0.05 | 0.30 |
| 1^{st} month | Largest single Impurity | 0.09 | 0.25 |
| | Total impurities | 0.20 | 0.53 |
| 2^{nd} month | Largest single Impurity | 0.15 | 0.36 |
| | Total impurities | 0.31 | 0.59 |
| 3^{rd} month | Largest single Impurity | 0.13 | 0.52 |
| | Total impurities | 0.28 | 0.81 |
| 6^{th} month | Largest single Impurity | 0.16 | 0.77 |
| | Total impurities | 0.41 | 1.24 |

The condition of the long-term stability test: the pharmaceutical compositions prepared in Example 7 and Comparative example 3 were respectively packaged according to a composite film for packaging a polyester/aluminum/polyethylene medicine product, and stood for 24 months under the temperature of 25°C and the relative humidity of 60%, sampling was performed in the 0^{th}, 1^{st}, 3^{rd}, 6^{th}, 12^{th}, 18^{th} and 24^{th} months during the test, 12-month stability test was completed at present, and inspection was performed according to stability investigation items, where the inspection results of long-term test of "related substances" are shown in Table 3.

**Table 3 Inspection results of related substances of long-term stability test**

| Inspection Items | | Related Substances (%) | |
|---|---|---|---|
| | | Example 7 | Comparative example 3 |
| 0^{th} month | Largest single Impurity | 0.05 | 0.07 |
| | Total impurities | 0.05 | 0.30 |
| 3^{rd} month | Largest single Impurity | 0.10 | 0.07 |
| | Total impurities | 0.15 | 0.31 |
| 6^{th} month | Largest single Impurity | 0.10 | 0.09 |
| | Total impurities | 0.23 | 0.36 |
| 12^{th} month | Largest single Impurity | 0.14 | 0.24 |
| | Total impurities | 0.31 | 0.51 |

According to the stability investigation results of long-term and accelerated tests, Example 7 has higher stability compared with Comparative example 3. The related substances of the product may be affected by temperature and humidity, so the product should be stored in a cool place.

The above is merely illustrative of the preferred embodiments of the present application, the scope of which is defined by the appended claims.

## Claims

1. A pharmaceutical composition, wherein the pharmaceutical composition is a granule, and the granule comprises:
an active ingredient, comprising 2-(2-aminopyridine-4-yl)-5-(5-((5-(2-ethyl-2H-tetrazole-5-yl)pyridine-2-yl)-oxy)-3,3-amyldimethyl)-1,2,5-thiadiazolidin 1,1-dioxide or pharmaceutically acceptable salt thereof;
the pharmaceutical composition is **characterized by** further comprising auxiliary materials comprising a diluent, a suspending agent and a solubilizer;
the diluent comprising one or more of sucrose, mannitol, sorbitol and xylitol, the suspending agent comprising povidone, and the solubilizer comprising poloxamer 188 and/or polysorbate 80;
wherein, the active ingredient accounts for 1.0% to 3.0% of a total weight of the pharmaceutical composition, the diluent accounts for 60% to 98% of the total weight of the pharmaceutical composition, the suspending agent accounts for 0.3% to 2.0% of the total weight of the pharmaceutical composition, and the solubilizer accounts for 0.5% to 2.0% of a total weight of the pharmaceutical composition.

2. The pharmaceutical composition according to claim 1, wherein the suspending agent further comprises one or more of carboxymethylcellulose sodium, carrageenan or crospovidone (CL-M).

3. The pharmaceutical composition according to claim 1, wherein the pharmaceutically acceptable salt comprises hydrochloride, sulfate, citrate, benzenesulfonate, hydrobromide, hydrofluoric acid, phosphate, acetate, propionate, succinate, oxalate, malate, fumarate, maleate, tartrate or trifluoroacetate.

4. The pharmaceutical composition according to claim 1, wherein the povidone is povidone K30 or povidone K90.

5. The pharmaceutical composition according to claim 1, wherein the auxiliary materials further comprise essence; the essence comprises one or more of orange essence, strawberry essence and banana essence; and the essence preferably accounts ≤ 1.0% of a total weight of the pharmaceutical composition.

6. A method for preparing the pharmaceutical composition according to claim 1, comprising:
a) obtaining an adhesive by the suspending agent, the solubilizer and ethanol;
b) mixing the active ingredient and the diluent, and adding the adhesive for granulation;
c) performing dynamic drying; and
d) performing granule arrangement and total mixing.

7. The method according to claim 6, wherein the method comprises:
a) adding 0.4 parts of poloxamer 188 and 0.3 parts of povidone K90 to a 80% ethanol solution for uniform mixing to serve as the adhesive;
b) weighing and uniformly mixing 2-(2-aminopyridine-4-yl)-5-(5-((5-(2-ethyl-2H-tetrazole-5-yl)pyridine-2-yl)-oxy)-3,3-amyldimethyl)-1,2,5-thiadiazolidin 1,1-dioxide hydrochloride, mannitol, sorbitol and crospovidone (CL-M) according to a weight ratio of 1:26:12:0.4 by part, and then adding the adhesive to obtain a soft material, and performing sieving with a 841 micrometer (20-mesh) sieve for granulation:
c) performing drying at 50°C; and
d) performing granule arrangement with a 841 micrometer (20-mesh) sieve, adding essence to the granules after granule arrangement for total mixing, and packaging.

## Patentansprüche

1. Pharmazeutische Zusammensetzung, wobei die pharmazeutische Zusammensetzung ein Korn ist, wobei das Korn umfasst:
einen aktiven Inhaltsstoff, umfassend 2-(2-Aminopyridin-4-yl)-5-(5-((5-(2-ethyl-2H-tetrazol-5-yl)pyridin-2-yl)-oxy)-3,3-amyldimethyl)-1,2,5-thiadiazolidin-1,1-dioxid oder ein pharmazeutisch akzeptables Salz davon;
wobei die pharmazeutische Zusammensetzung **dadurch gekennzeichnet ist, dass** sie ferner Hilfsmaterialien umfasst, die ein Verdünnungsmittel, ein Suspensionsmittel und einen Lösungsvermittler umfassen;
wobei das Verdünnungsmittel eines oder mehrere von Sucrose, Mannitol, Sorbitol und Xylitol umfasst, das Suspensionsmittel Povidon umfasst und der Lösungsvermittler Poloxamar 188 und/oder Polysorbat 80 umfasst;
wobei der aktive Inhaltsstoff 1,0 Gew.-% bis 3,0 Gew.-% des Gesamtgewichts der pharmazeutischen Zusammensetzung ausmacht, das Verdünnungsmittel 60 Gew.-% bis 98 Gew.-% des Gesamtgewichts der pharmazeutischen Zusammensetzung ausmacht, das Suspensionsmittel 0,3 Gew.-% bis 2,0 Gew.-% des Gesamtgewichts der pharmazeutischen Zusammensetzung ausmacht und der Lösungsvermittler 0,5 Gew.-% bis 2,0 Gew.-% des Gesamtgewichts der pharmazeutischen Zusammensetzung ausmacht.

2. Pharmazeutische Zusammensetzung nach Anspruch 1, wobei das Suspensionsmittel ferner eines oder mehrere von Carboymethylcellulosenatrium, Carrageen oder Crospovidon (CL-M) umfasst.

3. Pharmazeutische Zusammensetzung nach Anspruch 1, wobei das pharmazeutisch akzeptable Salz Hydrochlorid, Sulfat, Citrat, Benzolsulfonat, Hydrobromid, Fluorwasserstoffsäure, Phosphat, Acetat, Propionat, Succinat, Oxalat, Malat, Fumarat, Maleat, Tartrat oder Trifluoracetat umfasst.

4. Pharmazeutische Zusammensetzung nach Anspruch 1, wobei das Povidon Povidon K30 oder Povidon K90 ist.

5. Pharmazeutische Zusammensetzung nach Anspruch 1, wobei die Hilfsmaterialien ferner Essenz umfassen; wobei die Essenz eines oder mehrere von Orangenessenz, Erdbeeressenz und Bananenessenz umfasst; und die Essenz vorzugsweise 1,0 Gew.-% des Gesamtgewichts der pharmazeutischen Zusammensetzung ausmacht.

6. Verfahren zum Herstellen der pharmazeutischen Zusammensetzung nach Anspruch 1, umfassend:
a) Erlangen eines Klebstoffs durch das Suspensionsmittel, den Lösungsvermittler und Ethanol;
b) Mischen des aktiven Inhaltsstoffs und des Verdünnungsmittels und Zugeben des Klebstoffs zur Granulation;
c) Durchführen dynamischer Trocknung; und
d) Durchführen von Kornanordnung und umfassendem Mischen.

7. Verfahren nach Anspruch 6, wobei das Verfahren umfasst:
a) Zusetzen von 0,4 Teilen Poloxamer 188 und 0,3 Teilen Povidon K90 zu einer 80 %igen Ethanollösung zum gleichmäßigen Mischen, um als der Klebstoff zu dienen;
b) Abwiegen und gleichmäßiges Mischen von 2-(2-Aminopyridin-4-yl)-5-(5-((5-(2-ethyl-2H-tetrazol-5-yl)pyridin-2-yl)-oxy)-3,3-amyldimethyl)-1,2,5-thiadiazolidin-1,1-dioxidhydrochlorid, Mannitol, Sorbitol und Crospovidon (CL-M) gemäß einem Gewichtsverhältnis von 1:26:12:0,4 nach Teile und dann Zusetzen des Klebstoffs, um ein weiches Material zu erlangen, und Durchführen von Sieben mit einem 841-Mikrometer(20-Mesh)-Sieb zur Granulation;
c) Durchführen von Trocknen bei 50 °C; und
d) Durchführen von Kornanordnung mit einem 841-Mikrometer(20-Mesh)-Sieb, Zusetzen von Essenz zu den Körnern nach der Kornanordnung zum umfassenden Mischen und Verpacken.

## Revendications

1. Composition pharmaceutique, dans laquelle la composition pharmaceutique est un granule, et le granule comprend :
un actif ingrédient comprenant de la 2-(2-aminopyridine-4-yl)-5-(5-((5-(2-éthyl-2H-tétrazole-5-yl)pyridine-2-yl)-oxy)-3,3-amyldiméthyl)-1,2,5-thiadiazolidine 1,1-dioxyde ou un sel pharmaceutiquement acceptable de celui-ci ;
la composition pharmaceutique est **caractérisée en ce qu'**elle comprend des matériaux auxiliaires comprenant un diluant, un agent de suspension et un solubilisant ;
le diluant comprenant un ou plusieurs éléments de saccharose, de mannitol, de sorbitol et de xylitol, l'agent de suspension comprenant la povidone et le solubilisant comprenant le poloxamère 188 et/ou le polysorbate 80 ;
dans lequel l'ingrédient actif représente 1,0 % à 3,0 % du poids total de la composition pharmaceutique, le diluant représente 60 % à 98 % du poids total de la composition pharmaceutique, l'agent de suspension représente 0,3 % à 2,0 % du poids total de la composition pharmaceutique et le solubilisant représente 0,5 % à 2,0 % du poids total de la composition pharmaceutique.

2. Composition pharmaceutique selon la revendication 1, dans laquelle l'agent de suspension comprend en outre un ou plusieurs composés de carboxyméthylcellulose sodique, de carraghénane ou de crospovidone (CL-M).

3. Composition pharmaceutique selon la revendication 1, dans laquelle le sel pharmaceutiquement acceptable comprend du chlorhydrate, du sulfate, du citrate, du benzènesulfonate, du bromure d'eau, de l'acide fluorhydrique, du phosphate, de l'acétate, du propionate, du succinate, de l'oxalate, du malate, du fumarate, du maléate, du tartrate ou du trifluoroacétate.

4. Composition pharmaceutique selon la revendication 1, dans laquelle la povidone est la povidone K30 ou la povidone K90.

5. Composition pharmaceutique selon la revendication 1, dans laquelle les matériaux auxiliaires comprennent en outre de l'essence ; l'essence comprend une ou plusieurs essences d'orange, de fraise et de banane ; et l'essence représente de préférence 1,0 % d'un poids total de la composition pharmaceutique.

6. Procédé de préparation de la composition pharmaceutique selon la revendication 1, comprenant :
a) l'obtention d'un adhésif par l'agent de suspension, le solubilisant et l'éthanol ;
b) le mélange de la matière active et du diluant, et ajout de l'adhésif pour la granulation ;
c) le séchage dynamique ; et
d) l'arrangement des granulés et le mélange total.

7. Procédé selon la revendication 6, dans lequel le procédé comprend :
a) l'ajout de 0,4 partie de poloxamère 188 et de 0,3 partie de povidone K90 à une solution d'éthanol à 80 % pour un mélange uniforme servant d'adhésif ;
b) la pesée et le mélange uniforme de chlorhydrate de 2-(2-aminopyridine-4-yl)-5-(5-((5-(2-éthyl-2H-tétrazole-5-yl)-pyridine-2-yl)-oxy)-3,3-amyldiméthyl)-1,2,5-thiadiazolidine 1,1-dioxyde de chlorhydrate, de mannitol, de sorbitol et de crospovidone (CL-M) selon un rapport de poids de 1:26:12:0,4 par partie, puis en ajoutant l'adhésif pour obtenir un matériau mou, et en effectuant un tamisage avec un tamis de 841 micromètres (maille de 20) pour la granulation :
c) le séchage à 50 °C ; et
d) l'arrangement des granules avec un tamis de 841 micromètres (maille de 20), en ajoutant de l'essence aux granules après l'arrangement des granules pour le mélange total et l'emballage.
